# EUROPEAN PATENT SPECIFICATION

(11) **EP 2 368 486 B1**
(45) Date of publication and mention of the grant of the patent: **20.03.2013**
(21) Application number: 11152075.5
(22) Date of filing: 25.01.2011
(51) Int. Cl.: A61B 1/04, A61B 5/1455

(54) **Electronic endoscope system**
Elektronisches Endoskopsystem
Système endoscopique électronique

(30) Priority: 23.03.2010 JP 2010066783
(43) Date of publication of application: 28.09.2011
(73) Proprietor: Fujifilm Corporation, Minato-ku Tokyo (JP)
(72) Inventor: Saito, Takaaki, Kanagawa (JP)
(74) Representative: Klunker . Schmitt-Nilson . Hirsch

(56) References cited:
- JP-A- 2002 085 344
- US-A- 4 878 113
- US-A1- 2006 184 037

## Description

### BACKGROUND OF INVENTION

The present invention relates to an electronic endoscope system for acquiring information on a blood vessel from an image acquired by an electronic endoscope and producing an image from the acquired information.

In recent years, a number of diagnoses and treatments using electronic endoscopes have been made in the field of medicine. A typical electronic endoscope is equipped with an elongated insertion section that is inserted into a subject's body cavity. The insertion section has therein incorporated an imager such as a CCD at the tip thereof. The electronic endoscope is connected to a light source device, which emits light from the tip of the insertion section to illuminate the inside of a body cavity. With the inside of the body cavity illuminated by light, the subject tissue inside the body cavity is imaged by an imager provided at the tip of the insertion section. Images acquired by imaging undergoes various kinds of processing by a processor connected to the electronic endoscope before being displayed by a monitor. Thus, the electronic endoscope permits real-time observation of images showing the inside of the subject's body cavity and thus enables sure diagnoses.

The light source device uses a white light source such as a xenon lamp capable of emitting white broadband light whose wavelength ranges from a blue region to a red region. Use of white broadband light to illuminate the inside of a body cavity permits observing the whole subject tissue from the acquired images thereof. However, although images acquired by broadband light illumination permit generally observing the whole subject tissue, there are cases where such images fail to enable clear observation of subject tissues such as micro-blood vessels, deep-layer blood vessels, pit patters, and uneven surface profiles formed of recesses and bumps. As is known, such subject tissues may be made clearly observable when illuminated by narrowband light having a wavelength limited to a specific range. As is also known, image data obtained by illumination with narrowband light yields various kinds of information on a subject tissue such as oxygen saturation level in a blood vessel.

However, images acquired by narrowband light illumination are light frame sequential images, which cannot be readily compared or combined because of the positional shift that occurs as time passes. JP 03-21186 A, JP 2001-218217 A, and JP 2002-85344 A propose methods of correcting such a positional shift between the frame sequential images.

For example, JP 03-21186 A describes displaying an oxygen saturation level image by acquiring images as the wavelengths are switched frame-sequentially, detecting the inter-frame color deviation, and, when the inter-frame color deviation is outside an allowance, adding information that a region of interest is an invalid region.

JP 2001-218217 A describes comparing monochromatic images, detecting the subject's movement amount based on a positional shift among images, and shifting images of the other colors.

JP 2002-85344 A describes binarizing frame sequential images by contour definition and calculating the barycentric coordinates of the binarized images, calculating the displacement from the barycenters of two monochromatic frame sequential images in order to synchronize the frame sequential images based on the displacement.

US 4,878,113 and US 2006/184037 disclose prior art systems according to the preamble of claim 1.

### SUMMARY OF INVENTIION

In recent years, there has been a demand for producing an oxygen saturation level image by aligning a plurality of frame images that are frame-sequentially acquired using light having different wavelengths based on the position of blood vessels. However, the method described in JP 03-21186 A fails to correct color deviation, if any, and therefore does not enable production of an accurately aligned image.

JP 2001-218217 A and JP 2002-85344 A make no mention of displaying blood vessel information functions such as oxygen saturation level. In addition, the inventions described therein do not base the alignment on the position of blood vessels and, therefore, could not produce an accurately aligned oxygen saturation level image.

The present invention has been made in view of the above problems and it is thus an object of the present invention is to accurately obtain an oxygen saturation level of a blood vessel, which is of great importance in diagnosis, by producing an oxygen saturation level image through alignment of a plurality of frame images that are frame-sequentially acquired using light of different wavelengths based on the position of blood vessels.

To achieve the above objects, the present invention provides an electronic endoscope system comprising: a light source device for sequentially emitting plural kinds of light having different wavelength bands from each other; an electronic endoscope for sequentially illuminating the plural kinds of light emitted from said light source device to a subject tissue containing blood vessels inside a body cavity, sequentially receiving the plural kinds of reflected light of the illuminated light from the subject tissue, and sequentially outputting image data corresponding to the plural kinds of received light having the different wavelength bands; a blood vessel extraction means for extracting positions of a blood vessel having a specified diameter from each of image data corresponding to the plural kinds of light having different wavelength bands outputted from said electronic endoscope; an alignment means for aligning images corresponding to the image data obtained using the plural kinds of light having different wavelength bands based on the positions of the blood vessel extracted by said blood vessel extraction means; an image production means for producing an oxygen saturation level image representing a distribution of an oxygen saturation level in the blood vessel from the image data of the images aligned by said alignment means; and an image display means for displaying in simulated color an oxygen saturation level image produced by said image production means.

Preferably, the light source device sequentially emits three kinds of light having the different wavelength bands, and the alignment means aligns, with respect to an image of image data obtained using a kind of light having an intermediate wavelength band of the three kinds of light having different wavelength bands, other images of image data obtained using two kinds of light having other wavelength bands of the three kinds of light.

Preferably, the light source device sequentially emits three kinds of light having different wavelength bands, the blood vessel extraction means extracts positions of a first blood vessel having a first diameter from first image data among the image data corresponding to the three kinds of light having different wavelength bands, extracts positions of the first blood vessel and positions of a second blood vessel having a second diameter greater than the first diameter from second image data among the image data, and extracts positions of the second blood vessel from third image data among the image data, and the alignment means aligns two images of the first and the second image data based on the positions of the first blood vessel, and aligns two images of the second and the third image data based on the positions of the second blood vessel.

Preferably, the light source device sequentially emits as a first light the three kinds of light having different wavelength bands and as a second light broadband light containing light having a whole wavelength band of the first light, and the alignment means aligns each of images of individual image data obtained using the first light with an image of image data obtained using the second light.

Preferably, the light source device sequentially emits three kinds of light having wavelengths of 540±10nm, 560±10nm and 580±10nm.

Preferably, the light source device sequentially emits three kinds of light having wavelengths of 405±10nm, 440±10nm and 470±10nm.

The present invention, provided both an alignment function based on a blood vessel position and an oxygen saturation level derivation function, permits accurately obtaining the oxygen saturation level of a blood vessel, which is of critical importance in diagnosis.

### BRIEF DESCRIPTION OF DRAWINGS

Fig. 1 is an external view of an electronic endoscope system according to a first embodiment of the invention.
Fig. 2 is a block diagram illustrating an electric configuration of the electronic endoscope system according to the first embodiment of the invention.
Fig. 3 is a schematic view of a rotary filter.
Fig. 4 is a view for explaining imaging operations of a CCD (image sensor) according to the first embodiment of the invention.
Fig. 5 is a graph illustrating an absorption coefficient of hemoglobin.
Fig. 6 is a graph illustrating a correlation between first and second luminance ratios S1/S3 and S2/S3 on the one hand and blood vessel depth and oxygen saturation level on the other hand.
Fig. 7A is a view for explaining how a coordinate point (X*, Y*) in a luminance coordinate system is obtained from the first and the second luminance ratios S1*/S3* and S2*/S3*; Fig. 7B is a view for explaining how a coordinate point (U*, V*) in a blood vessel information coordinate system corresponding to the coordinate point (X*, Y*) is obtained.
Fig. 8 is a view of a monitor screen displaying a narrowband image, a broadband image, or an oxygen saturation level image.
Fig. 9 is a view of a monitor screen displaying both an oxygen saturation level image and a narrowband image or a broadband image simultaneously.
Fig. 10 is a flow chart illustrating the first half of a procedure of calculating a blood vessel depth-oxygen saturation level information and a procedure of producing oxygen saturation level image incorporating that information.
Fig. 11 is the second half of the flow chart illustrating a procedure following the procedure of calculating blood vessel depth-oxygen saturation level information and a procedure of producing oxygen saturation level information incorporating that information.
Fig. 12 is a block diagram illustrating an electric configuration of the electronic endoscope system according to a second embodiment of the invention.
Fig. 13 is a block diagram illustrating an electric configuration of the electronic endoscope system according to a third embodiment of the invention.
Fig. 14 is a graph illustrating spectral transmittances of red, green, and blue filters.

### DETAILED DESCRIPTION OF INVENTION

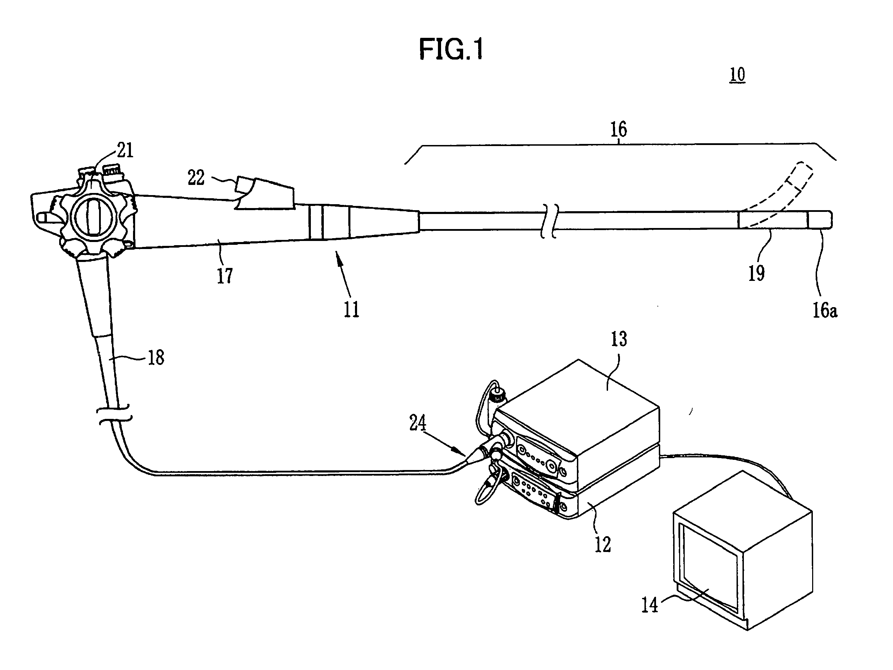

As illustrated in Fig. 1, an electronic endoscope system 10 according to the first embodiment of the invention comprises an electronic endoscope 11 for imaging the inside of a subject's body cavity, a processor 12 for producing an image of a subject tissue in the body cavity based on a signal acquired by imaging, a light source device 13 for supplying light used to illuminate the inside of the body cavity, and a monitor (image display means) 14 for displaying the image of the inside of the body cavity. The electronic endoscope 11 comprises a flexible insertion section 16 that is inserted into a body cavity, an operating section 17 provided at the base of the insertion section 16, and a universal cord 18 for connecting the operating section 17 to the processor 12 and the light source device 13.

The insertion section 16 has a bending portion 19 at the tip thereof comprising connected bending pieces. The bending portion 19 bends up and down, left and right in response to the operation of an angle knob 21 of the operating section 17. The bending portion 19 has at its tip a leading end portion 16a incorporating an optical system and other components for imaging the inside of a body cavity. The leading end portion 16a can be directed in a desired direction in the body cavity according to a bending operation of the bending portion 19.

The universal cord 18 has a connector 24 provided on the side thereof leading to the processor 12 and the light source device 13. The connector 24 is a composite type connector composed of a communication connector and a light source connector and removably connects the electronic endoscope 11 to the processor 12 and the light source device 13 through the connector 24.

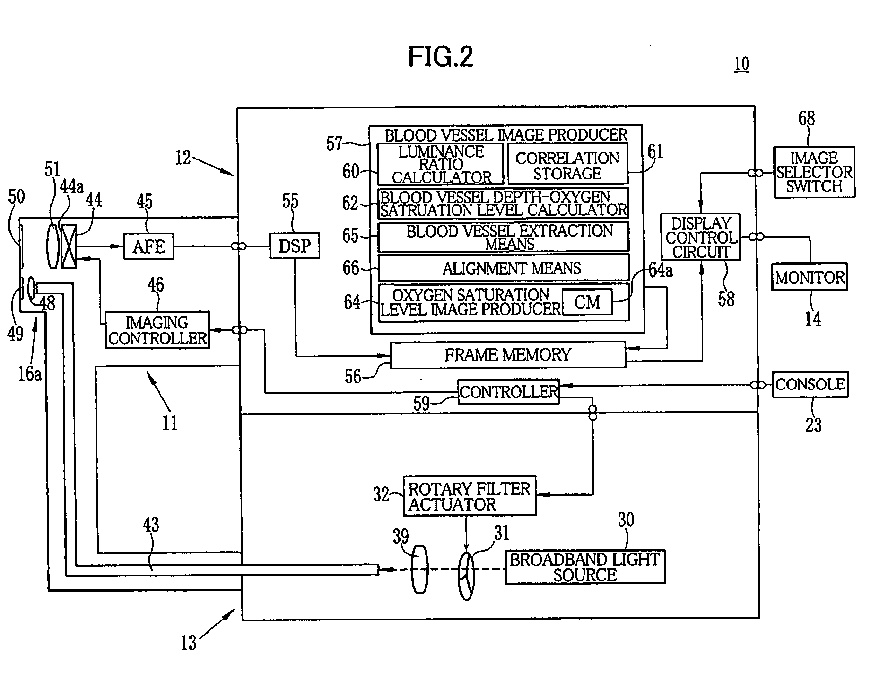

As illustrated in Fig. 2, the light source device 13 comprises a broadband light source 30, a rotary filter 31, a rotary filter actuator 32, and a condenser lens 39. The broadband light source 30 is a xenon lamp, a white LED, a Micro White (trademark) light source, or the like and produces broadband light BB having a wavelength ranging from a blue region to a red region (about 470 nm to 700 nm). The broadband light BB emitted from the broadband light source 30 enters the rotary filter 31, leaves the rotary filter 31 as narrowband light having a specified bandwidth and is focused by the condenser lens 39 before entering a light guide 43.

The rotary filter 31 is provided between the broadband light source 30 and the condenser lens 39 and receives the broadband light BB to pass only narrowband light having a given bandwidth corresponding to a transmission region of the rotary filter 31.

The rotary filter actuator 32 is connected to a controller 59 in the processor and controls the position of the transmission regions of the rotary filter 31 by turning the rotary filter 31 in response to an instruction from the controller 59.

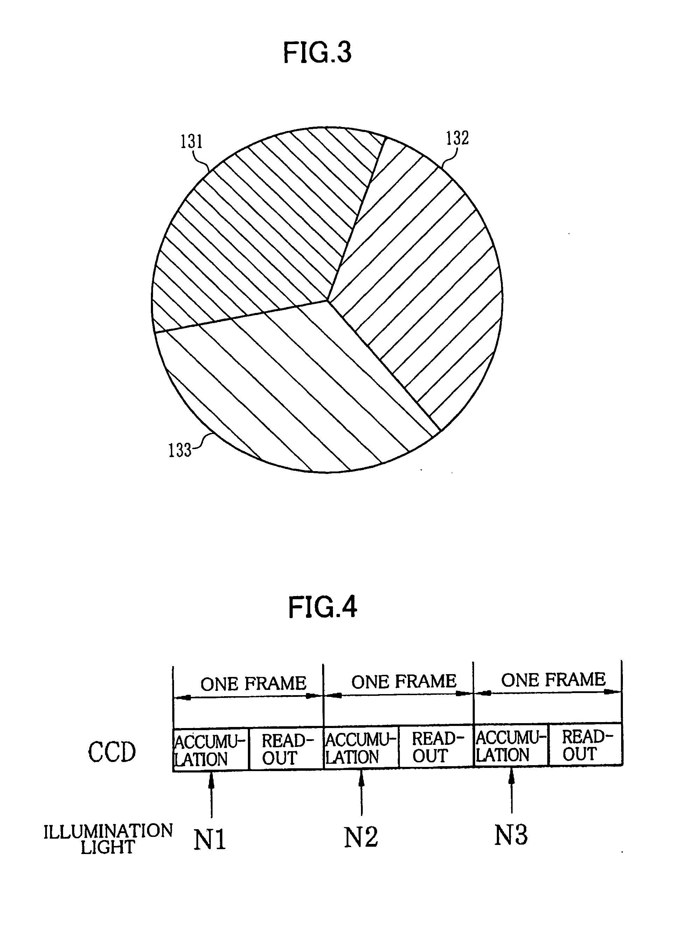

The rotary filter 31 illustrated in Fig. 3 comprises a first narrowband light transmission region 131 for passing, of the broadband light BB, narrowband light having a wavelength limited to 540 nm +/- 10 nm, preferably 540 nm (referred to below as "first narrowband light N1"), a second narrowband light transmission region 132 for passing narrowband light having a wavelength limited to 560 nm +/- 10 nm, preferably 560 nm (referred to below as "second narrowband light N2"), and a third narrowband light transmission region 133 for passing narrowband light having a wavelength limited to 580 nm +/- 10 nm, preferably 580 nm (referred to below as "third narrowband light N3").

The rotary filter 31 is capable of rotation and, in order to produce one of the first narrowband light N1 to N3 from the light source device 13, it is turned so as to position one of the broadband light transmission regions 131 to 133 corresponding to that narrowband light to be produced on the optical path of the broadband light source 30.

Specifically, in response to an instruction from the controller 59, the rotary filter actuator 32 turns the rotary filter 31 to position the first narrowband light transmission region 131 on the optical path of the broadband light source 30. With the first narrowband light N1 illuminating the inside of a body cavity, a subject tissue is imaged. Upon completion of imaging, the controller 59 gives an instruction for turning the filter to set the second narrowband light transmission region 132 in position in lieu of the first narrowband light transmission region 131. Upon completion of imaging with the second narrowband light N2 illuminating the inside of the body cavity, the third narrowband light transmission region 133 is likewise set in position in lieu of the second narrowband light transmission region 132. Upon completion of imaging with the third narrowband light N3 illuminating the inside of the body cavity, the first narrowband light transmission region 131 is set back in position again in lieu of the third narrowband light transmission region 133.

The electronic endoscope 11 comprises a light guide 43, a CCD 44 (image sensor), an analog processing circuit (AFE: analog front end) 45, and an imaging controller 46. The light guide 43 is a large-diameter fiber, a bundle fiber, or the like having its light receiving end inserted in the light source device and its light emitting end facing an illumination lens 48 provided at the leading end portion 16a. The light emitted by the light source device 13 is guided by the light guide 43 and emitted toward the illumination lens 48. The light admitted in the illumination lens 48 passes through an illumination window 49 attached to the end face of the leading end portion 16a to enter the body cavity. The broadband light BB and the first to the third narrowband light N1 to N3 reflected by the inside of the body cavity pass through an observation window 50 attached to the end face of the leading end portion 16a to enter a condenser lens 51.

The CCD 44 receives the light from the condenser lens 51 with its imaging surface 44a, performs photoelectric conversion of the received light to accumulate a signal charge, and reads out the accumulated signal charge as an imaging signal. The read-out imaging signal is transmitted to an AFE 45. According to this embodiment, the CCD44 may be a color CCD or a monochromatic CCD.

The AFE 45 comprises a correlated double sampling (CDS), an automatic gain control circuit (AGC), and an analog-to-digital convertor (A/D) (none of them are shown). The CDS performs correlated double sampling of an imaging signal supplied from the CCD 44 to remove noise generated by actuation of the CCD 44. The AGC amplifies an imaging signal from which noise has been removed by the CDS. The analog-to-digital converter converts an imaging signal amplified by the AGC into a digital imaging signal having a given number of bits, which is applied to the processor 12.

The imaging controller 46 is connected to the controller 59 in the processor 12 and sends a drive signal to the CCD 44 in response to an instruction given by the controller 59. The CCD 44 outputs an imaging signal to the AFE 45 at a given frame rate according to the drive signal from the imaging controller 46.

According to the first embodiment, a total of two operations are first performed in one frame of acquisition period as illustrated in Fig. 4: a step of accumulating a signal charge through photoelectric conversion of the first narrowband light N1 and a step of reading out the accumulated signal charge as a first narrowband imaging signal. Upon completion of readout of the first narrowband imaging signal, a step of accumulating a signal charge through photoelectric conversion of the second narrowband light N2 and a step of reading out the accumulated signal charge as a second narrowband imaging signal are performed in one frame of acquisition period. Upon completion of readout of the second narrowband imaging signal, a step of accumulating a signal charge through photoelectric conversion of the third narrowband light N3 and a step of reading out the accumulated signal charge as a third narrowband imaging signal are performed in one frame of acquisition period.

As illustrated in Fig. 2, the processor 12 comprises a digital signal processor 55 (DSP), a frame memory 56, a blood vessel image producer 57, and a display control circuit 58, all of these components being controlled by the controller 59. The DSP 55 performs color separation, color interpolation, white balance adjustment, gamma correction, and the like of the first to the third narrowband imaging signals produced from the AFE 45 of the electronic endoscope to produce the first to the third narrowband image data. The frame memory 56 stores the first to the third narrowband image data produced by the DSP 55.

The blood vessel image producer 57 comprises a luminance ratio calculator 60, a correlation storage 61, a blood vessel depth-oxygen saturation level calculator 62, an oxygen saturation level image producer 64, a blood vessel extraction means 65, and an alignment means 66.

The blood vessel extraction means 65 performs blood vessel extraction in the first to the third narrowband image data obtained by the CCD 44.

Because the hemoglobin light absorption characteristics and the digestive tract mucosa scattering characteristics are the same among the first to the third narrowbands according to this embodiment, the configuration of a blood vessel observed using the first to the third narrowband light does not significantly vary. To be more specific, a blood vessel lying in a layer (intermediate layer) that is slightly deeper than a superficial layer of the mucous membrane (lying in a depth of about 100 µm) is extracted with a highest contrast. Therefore, a signal having a frequency component corresponding to a blood vessel lying in an intermediate layer and having a diameter of about 20 µm to 50 µm is extracted.

A frequency signal corresponding to such a blood vessel lying in an intermediate layer can be extracted using, for example, a specified two-dimensional filter.

To produce such a two-dimensional filter, first the frequency band in an image corresponding to the diameter of the blood vessel in the intermediate layer (measuring 20µm to 50µm in diameter) is obtained by estimating a distance and a magnification ratio between the leading end portion 16a of the endoscope and the subject. Next, a filter that intensifies only that frequency band is designed in frequency space and then adapted to correspond to real space through Fourier transformation. In the present case, the two-dimensional filter characteristics need to be adjusted in frequency space so that the size of the two-dimensional filter can be contained within a realistic size of say about 5 x 5.

The two-dimensional filter thus produced is applied to the first to the third narrowband image data to extract data of a frequency component corresponding to the blood vessel in the intermediate layer.

Thus, application of the two-dimensional filter results in extraction of data of a frequency component corresponding to the blood vessel in the intermediate layer in the first to the third narrowband image data.

The image data where the component corresponding to the blood vessel has been extracted in the first to the third narrowband image data will be referred to as first to third intensified narrowband image data. The first to the third intensified narrowband image data are stored in the frame memory 56.

The pixel position of the pixel representing the blood vessel in the intermediate layer in the first to the third narrowband image data is determined as a blood vessel region through the blood vessel extraction processing and stored as such in the frame memory 56 together with the first to the third narrowband image data.

The alignment means 66 reads out the first to the third intensified narrowband image data stored in the frame memory 56 to achieve alignment of the first to the third intensified narrowband image data based on the intensified blood vessel image.

According to this embodiment, the first and the third intensified narrowband images are aligned with the second intensified narrowband image taking into consideration the order in which they are acquired because the positional shift (movement amount) to be corrected then amounts to only one frame.

Alignment may be achieved using the method described in JP 2001-218217 A.

First, the first intensified narrowband image is shifted by several pixels up- and downward and left- and rightward to obtain a difference from the second intensified narrowband image. This process is repeated a plurality of times to determine a shift amount minimizing the sum of absolute values of the differential signals between the pixels. Then the first narrowband image is moved by the same shift amount as that shift amount thus determined to obtain a first moved narrowband image, of which the image data is stored in the frame memory 56.

The same process is repeated with the third intensified narrowband image and the third narrowband image to obtain a third moved narrowband image, of which the image data is stored in the memory 56.

The luminance ratio calculator 60 reads out the first and the third moved narrowband image data and the second narrowband image data stored in the frame memory 56 and calculates luminance ratios between the images.

Because the first and the third moved narrowband image data and the second narrowband image data are in alignment achieved by the alignment means 66 based on the blood vessel (about 20µm to 50µm in diameter), the pixel position and the blood vessel position coincide among these image data.

The luminance ratio calculator 60 obtains a first luminance ratio S1/S3 between the first and the third moved narrowband image data and a second luminance ratio S2/S3 between the second narrowband image data and the third moved narrowband image data corresponding to a pixel at the same position in the blood vessel region. S1 is a luminance of a pixel of the first moved narrowband image data, S2 a luminance of a pixel of the second narrowband image data, and S3 a luminance of a pixel of the third moved narrowband image data.

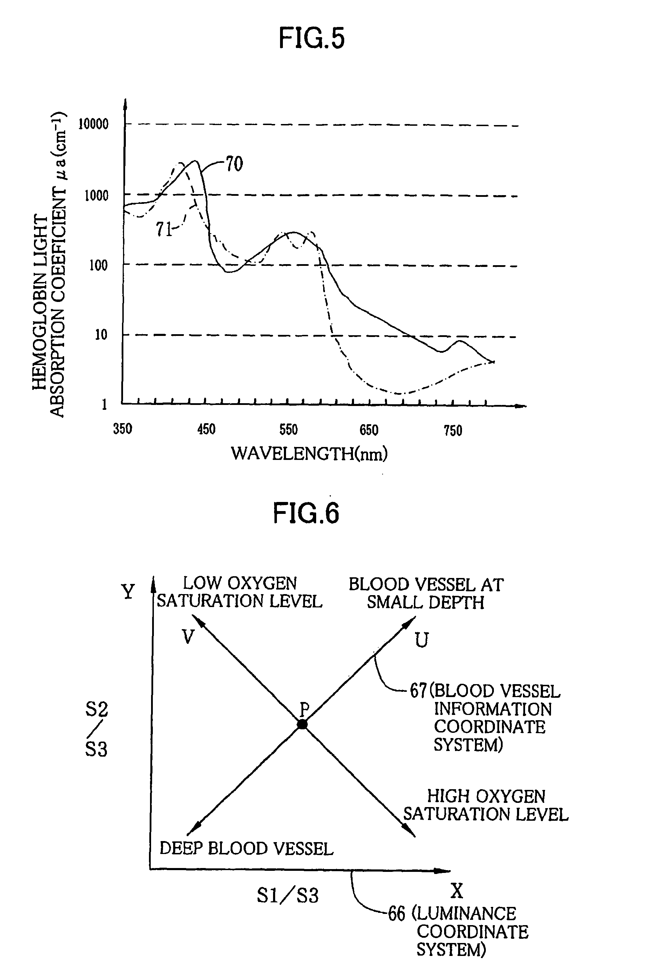

The correlation storage 61 stores a correlation between the first and the second luminance ratios S1/S3 and S2/S3 on the one hand and an oxygen saturation level in a blood vessel and a blood vessel depth on the other hand. That correlation is one where a blood vessel contains hemoglobin exhibiting light absorption coefficients as shown in Fig. 5 and is obtained by analyzing, for example, a number of the first to the third narrowband image data accumulated through diagnoses hitherto made. As illustrated in Fig. 5, the hemoglobins in a blood vessel have light absorptions characteristics having the light absorption coefficient µa changing according to the wavelength of light used for illumination. The light absorption coefficient µa indicates an absorbance, i.e., a degree of light absorption by hemoglobin, and is a coefficient in an expression I0exp (-µa×x) showing the attenuation of light illuminating the hemoglobin. In this expression, Io is the intensity of light emitted from the light source device to illuminate a subject tissue; x (cm) is a depth of a blood vessel inside the subject tissue.

A reduced hemoglobin 70 and an oxygenated hemoglobin 71 have different light absorption characteristics such that they have different absorbances except for the isosbestic point at which both exhibit the same absorbance (intersection of light absorption characteristics curves of hemoglobins 70 and 71 in Fig. 5). With a difference in absorbance, the luminance varies even when the same blood vessel is illuminated by light having the same intensity and the same wavelength. The luminance also varies when the illumination light has the same intensity but varies in wavelength because a difference in wavelength causes the light absorption coefficient µa to change.

In view of the light absorption characteristics of hemoglobin as described above and considering the fact that wavelengths whereby the absorbance varies according to the oxygen saturation level lie in a range of of 445 nm and 504 nm and that light having a short wavelength and hence having a short reaching depth is required in order to retrieve blood vessel depth information, at least one of the first to the third narrowband light N1 to N3 preferably has a wavelength range whose central wavelength is 450 nm or less. Further, with the same oxygen saturation level, a difference in wavelength causes a difference in absorption coefficient and also a difference in reaching depth into a mucus membrane. Therefore, using the property of light whose reaching depth varies with the wavelength permits obtaining correlation between luminance ratio and blood vessel depth.

As illustrated in Fig. 6, the correlation storage 61 stores a correlation in correspondence between the coordinate points in a luminance coordinate system 66 representing the first and the second luminance ratios S1/S3 and S2/S3 and the coordinate points in a blood vessel information coordinate system 67 representing oxygen saturation level and blood vessel depth. The luminance coordinate system 66 is an XY coordinate system, where the X axis shows the first luminance ratio S1/S3 and the Y axis shows the second luminance ratio S2/S3. The blood vessel information coordinate system 67 is a UV coordinate system provided on the luminance coordinate system 66, where the U axis shows the blood vessel depth and the V axis shows the oxygen saturation level. Because the blood vessel depth has a positive correlation with the luminance coordinate system 66, the U axis has a positive slope. The U axis shows that a blood vessel of interest is located at an increasingly smaller depth as a position on the U axis moves obliquely up rightward and that a blood vessel of interest is located at an increasingly greater depth as a position on the U axis moves obliquely down

leftward. Because the oxygen saturation level has a negative correlation with the luminance coordinate system 66, the V axis has a negative slope. The V axis shows that the oxygen saturation level is lower as a position on the V axis moves obliquely up leftward and that the oxygen saturation level is higher as a position on the V axis moves obliquely down rightward.

In the blood vessel information coordinate system 67, the U axis and the V axis cross each other at right angles at an intersection P. This is because the magnitude of absorbance reverses between illumination by the first narrowband light N1 and illumination by the second narrowband light N2. More specifically, as illustrated in Fig. 5, illumination by the first narrowband light N1 having a wavelength of 540 nm +/- 10 nm allows the light absorption coefficient of the oxygenated hemoglobin 71 having a high oxygen saturation level to be greater than the light absorption coefficient of the reduced hemoglobin 70 whereas illumination by the second narrowband light N2 having a wavelength of 560 nm +/- 10 nm allows the light absorption coefficient of the reduced hemoglobin 70 to be greater than the light absorption coefficient of the oxygenated hemoglobin 71, thus causing the magnitude of the absorbance to reverse.

When narrowband light permitting no absorbance reversal are used in lieu of the first to the third narrowband light N1 to N3, the U axis and the V axis do not cross each other at right angles.

The blood vessel depth-oxygen saturation level calculator 62 determines an oxygen saturation level and a blood vessel depth corresponding to the first and the second luminance ratios S1/S3 and S2/S3 calculated by the luminance ratio calculator 60 based on the correlation stored in the correlation storage 61. Now, in the first and the second luminance ratios S1/S3 and S2/S3 calculated by the luminance ratio calculator 60, let S1*/S3* and S2*/S3* be the first luminance ratio and the second luminance ratio respectively for a given pixel in the blood vessel region.

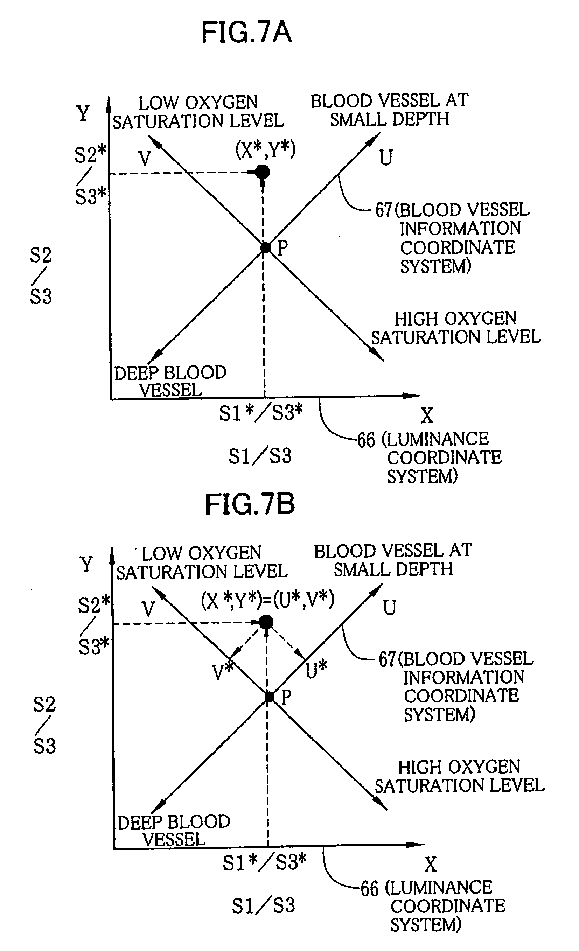

As illustrated in Fig. 7A, the blood vessel depth-oxygen saturation level calculator 62 determines a coordinate point (X*, Y*) corresponding to the first and the second luminance ratios S1*/S3* and S2*/S3* in the luminance coordinate system 66. Upon the coordinate point (X*, Y*) being determined, the blood vessel depth-oxygen saturation level calculator 62 determines a coordinate point (U*, V*) corresponding to the coordinate point (X*, Y*) in the blood vessel information coordinate system 67 as illustrated in Fig.7B. Thus, blood vessel depth information U* and oxygen saturation level information V* are obtained for a given pixel in the blood region.

The oxygen saturation level image producer 64 has a color map 64a (CM) where oxygen saturation levels are assigned color information. More specifically, the color map 64a permits easy distinction of oxygen saturation level by color assignment (simulated color assignment) such that, for example, a low oxygen saturation level is assigned a color of cyan, a medium oxygen saturation level is assigned a color of magenta, and a high oxygen saturation level is assigned a color of yellow. Similarly to the blood vessel depth image producer, the oxygen saturation level image producer 64 determines from the color map 64a color information corresponding to the oxygen saturation level information V* calculated by the blood vessel depth-oxygen saturation level calculator. Then, the oxygen saturation level image producer 64 incorporates this color information in specified narrowband image data or broadband image data to produce the oxygen saturation level image data in which the oxygen saturation level is displayed using simulated color, false color, or pseudo color. The oxygen saturation level image data thus produced is stored in the frame memory 56.

The display control circuit 58 reads out one or more images from the frame memory 56 and allows the monitor 14 to display the read-out image or images. The images may be displayed in various modes.

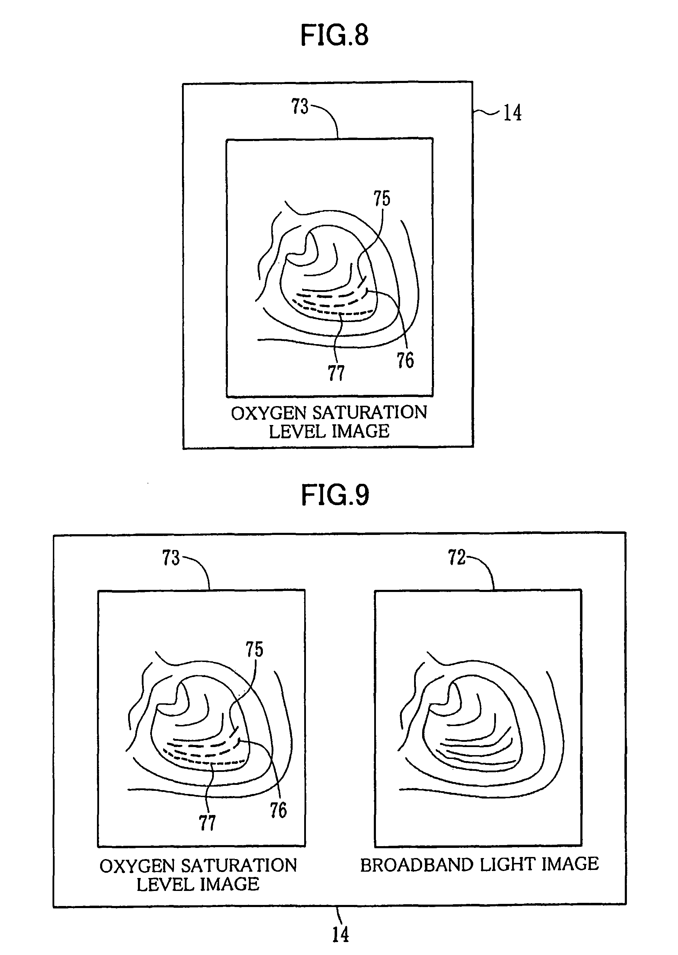

For example, as illustrated in Fig. 8, an oxygen saturation level image 73 may be simply displayed on the monitor 14. In the oxygen saturation level image 73, a blood vessel image 75 is shown in cyan indicating a lower oxygen saturation level, a blood vessel image 76 is shown in magenta indicating a medium oxygen saturation level, and a blood vessel image 77 is shown in yellow indicating a higher oxygen saturation level.

As illustrated in Fig. 9, both the oxygen saturation level image 73 and a narrowband image 72 or a broadband image 74 may be both displayed simultaneously with the display mode shown in Fig. 8.

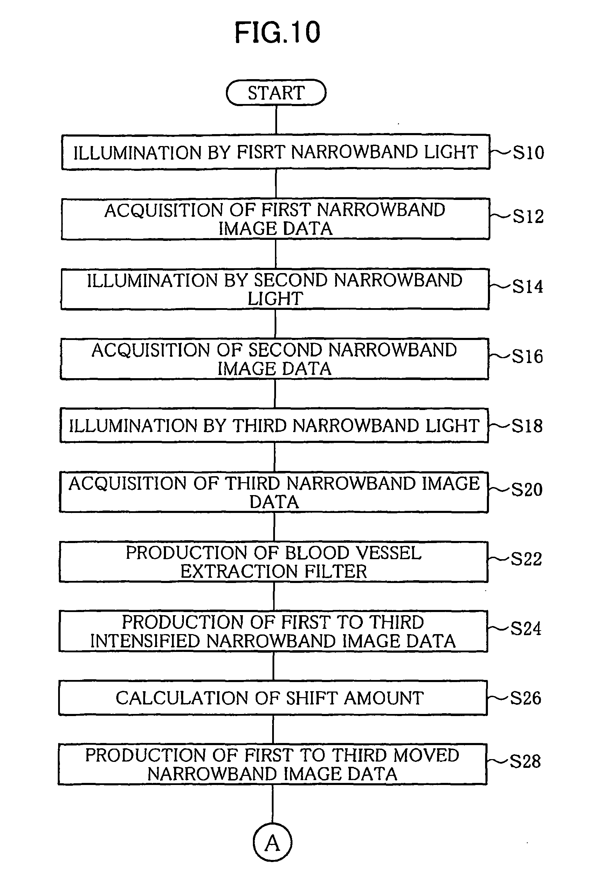

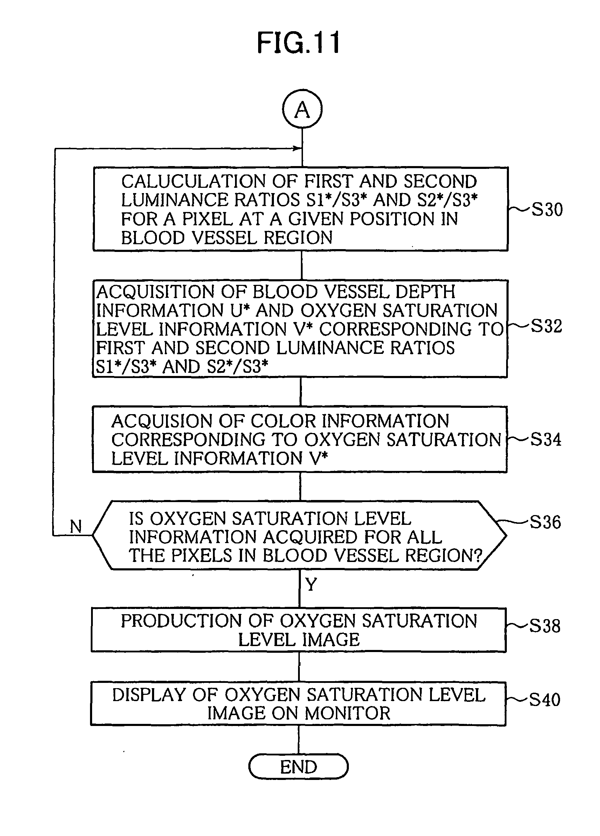

Next, reference is made to the flowchart illustrated in Figs. 10 and 11 to explain a procedure of acquiring the first to the third narrowband image data, a procedure of aligning the first to the third narrowband images, a procedure of calculating the blood vessel depth-oxygen saturation level information, and a procedure of producing an oxygen saturation level image incorporating these information. First, the console 23 is operated to cause the rotary filter actuator 32 to turn the rotary filter 31 so as to position the first narrowband light transmission region 131 on the optical path of the broadband light source 30. Then, imaging of the subject tissue is started with the first narrowband light N1 illuminating the inside of the body cavity. The first narrowband image observed in that imaging is stored in the frame memory 56 as the first narrowband image data.

Likewise, as illustrated in the flowchart, the rotary filter actuator 32 turns the rotary filter 31, the second narrowband light transmission region 132 and the third narrowband light transmission region 133 are set on the optical path of the broadband light source 30, and the inside of the body cavity is illuminated by the second narrowband light N2 and the third narrowband light N3 to image a subject tissue and store the second narrowband image and the third narrowband image in the frame memory 56 as the second narrowband image data and the third narrowband image data, respectively.

Upon storage of the first to the third narrowband image data in the frame memory 56, the blood vessel extraction means 65 produces a blood vessel extraction filter based on the diameter of a blood vessel to be extracted.

The blood vessel extraction filter first estimates a distance and a magnification ratio between the leading end portion 16a of the endoscope and the subject to obtain frequency bands in the first to the third narrowband images corresponding to the diameter of the blood vessel in the intermediate layer (measuring 20µm to 50µm in diameter). Next, a two-dimensional filter that intensifies only those frequency bands is designed in frequency space and then made to correspond to real space through Fourier transformation. In the present case, the filter characteristics need to be adjusted in frequency space so that the size of the two-dimensional filter can be contained within a realistic size of say about 5 x 5.

Application of the two-dimensional filter thus produced to the first to the third narrowband image data permits extraction of a blood vessel having a diameter of about 20 µm to 50 µm.

Then the first to the third narrowband image data where the blood vessel has been extracted are stored in the frame memory 56 as first to third intensified narrowband image data.

Next, the alignment means 66 reads out the first to the third intensified narrowband image data from the frame memory 56 to align the images.

The alignment is achieved taking into consideration the order in which they are acquired by using the second intensified narrowband image as reference so that the first and the third intensified narrowband images are aligned with the second intensified narrowband image

Specifically, the first intensified narrowband image is aligned first. The first intensified narrowband image is shifted by several pixels up- and downward and left- and rightward to obtain differences from the first narrowband image. This process is repeated a plurality of times to obtain a shift amount that minimizes the sum of absolute values of the differential signals between the pixels.

Then, the first narrowband image is read out from the frame memory 56 and moved by the same shift amount as the above shift amount to obtain a first moved narrowband image and store the first moved narrowband image data in the frame memory 56.

The third narrowband image is also aligned to obtain a third moved narrowband image, which is stored in the frame memory 56.

The first and the third moved narrowband image data and the second narrowband image data are used to produce the oxygen saturation level image.

When the first to the third moved narrowband image data and the second narrowband image data have been stored in the frame memory 56, the luminance ratio calculator 60 determines the blood vessel region containing a blood vessel from the three image data. Then, the luminance ratio calculator 60 calculates the first luminance ratio S1*/S3* between the first and the third moved narrowband image data and the second luminance ratio S2*/S3* between the second narrowband image data and the third moved narrowband image data corresponding to a pixel at the same position in the blood vessel region.

Next, the blood vessel depth-oxygen saturation level calculator 62 determines the coordinate point (X*, Y*) in the luminance coordinate system corresponding to the first and the second luminance ratios S1*/S3* and S2*/S3* based on the correlation stored in the correlation storage 61. Further, the coordinate point (U*, V*) in the blood vessel information coordinate system corresponding to the coordinate point (X*, Y*) is determined to obtain the blood vessel depth information U* and the oxygen saturation level information V* for a given pixel in the blood vessel region.

When the blood vessel depth information U* and the oxygen saturation level information V* have been obtained, color information corresponding to the blood vessel depth information U* is determined from the CM 63a in the blood vessel depth image producer while color information corresponding to the oxygen saturation level information V* is determined from the CM 64a in the oxygen saturation level image producer. The color information thus determined are stored in the RAM (not shown) in the processor 12.

Upon storage of the color information in the RAM, the above procedure is followed to obtain the blood vessel depth information U* and the oxygen saturation level information V* for all the pixels in the blood vessel region and determine color information corresponding to the blood vessel depth information U* and the oxygen saturation level information V*.

When the color information corresponding to the blood vessel depth information and the oxygen saturation level information for all the pixels in the blood vessel region have been obtained, the oxygen saturation level image producer 64 produces oxygen saturation level image data. The oxygen saturation level image producer 64 adds oxygen saturation level information to the image data of the image used as alignment reference (second narrowband image data). The oxygen saturation level image data thus produced is stored again in the frame memory 56.

The display control circuit 58 reads out a plurality of image data including the oxygen saturation level image data from the frame memory 56 and displays the oxygen saturation level image 73 and the narrowband image 72 or the broadband image 74 as illustrated in Fig. 8 or Fig. 9 on the monitor 14 based on those read-out image data.

As described above, the present invention provides both an alignment function based on a blood vessel position and an oxygen saturation level derivation function and permits accurately obtaining the oxygen saturation level of a blood vessel, which is of critical importance in diagnosis.

We have described above the first embodiment of the invention.

The second embodiment of the invention is provided with a narrowband light source producing narrowband light having a given wavelength in lieu of the broadband light source 30 such as a xenon light source.

The second embodiment differs from the first embodiment in the configuration of the light source 13, the blood vessel extraction processing by the blood vessel extraction means 65, and the alignment by the alignment means 66. The second embodiment share the other features with the first embodiment, and their descriptions therefore will not be repeated below.

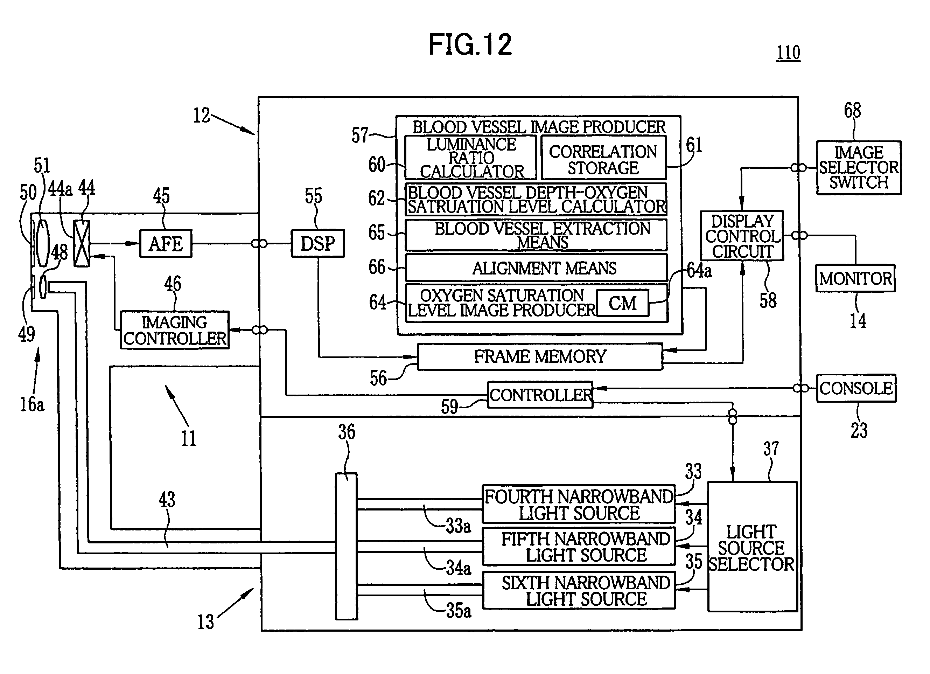

As illustrated in Fig. 12, the light source device 13 comprises fourth to sixth narrowband light sources 33 to 35, a coupler 36, and a light source selector 37.

The fourth to the sixth narrowband light sources 33 to 35 are laser diodes or the like. The fourth narrowband light source 33 produces narrowband light having a wavelength limited to 400 nm +/- 10 nm, preferably 405 nm (referred to below as "fourth narrowband light N4"), the fifth narrowband light source 34 produces narrowband light having a wavelength limited to 440 nm +/- 10 nm, preferably 445 nm (referred to below as "fifth narrowband light N5"), and the sixth narrowband light source 35 produces narrowband light having a wavelength limited to 470 nm +/- 10 nm, preferably 473 nm (referred to below as "sixth narrowband light N6"). The fourth to the sixth narrowband light sources 33 to 35 are connected respectively to fourth to the sixth narrowband optical fibers 33a to 35a, allowing the fourth to the sixth narrowband light N4 to N6 to enter the fourth to the sixth narrowband optical fibers 33a to 35a.

The coupler 36 connects a light guide 43 in the electronic endoscope to the fourth to the sixth narrowband optical fibers 33a to 35a. The fourth to the sixth narrowband light N4 to N6 can enter the light guide 43 through the fourth to the sixth narrowband optical fibers 33a to 35a.

The light source selector 37 is connected to the controller 59 in the processor and turns on or off the fourth to the sixth narrowband light sources 33 to 35 according to an instruction by the controller 59. According to the second embodiment, the fourth to the sixth narrowband light sources 33 to 35 are sequentially turned on to permit imaging using the fourth to the sixth narrowband light N4 to N6.

Specifically, the light source selector 37 first turns on the fourth narrowband light source 33. With the fourth narrowband light N4 illuminating the inside of a body cavity, a subject tissue is imaged. Upon completion of imaging, the controller 59 gives a light source switching instruction to turn off the fourth narrowband light source 33 and turn on the fifth narrowband light source 34. Likewise, upon completion of imaging with the fifth narrowband light N5 illuminating the inside of the body cavity, the fifth narrowband light source 34 is turned off and the sixth narrowband light source 35 is turned on. Upon completion of imaging with the sixth narrowband light N6 illuminating the inside of the body cavity, the sixth narrowband light source 35 is turned off.

Similarly to the first embodiment, the fourth narrowband light N4 to the sixth narrowband light N6 enter the CCD 44 of the endoscope 11 to produce a fourth narrowband imaging signal to a sixth narrowband imaging signal.

As in the first embodiment, the CCD 44 may be a color CCD or a monochromatic CCD according to this second embodiment.

Because the hemoglobin light absorption characteristics and the digestive tract mucosa scattering characteristics greatly vary among the three narrowband light N4 to N6, the configuration of a blood vessel observed greatly varies among the three wavelengths depending on the wavelength used.

Specifically, the fourth narrowband light N4 allows superficial-layer blood vessels having a diameter of 20 µm to 50 µm to be observed; the fifth narrowband light N5 allows both superficial-layer blood vessels and intermediate-layer blood vessels (whose surface lying in a depth greater than 100 µm and having a diameter of about 20 µm to 50 µm) to be observed, the superficial-layer blood vessels being observed with a higher contrast; and the sixth narrowband light N6 allows only the intermediate-layer blood vessels to be observed with a high contrast.

Thus, because the wavelengths of the narrowband light used for illumination and the blood vessel images vary between the first and the second embodiments, the blood vessel extraction processing according to the second embodiment also varies from that according to the first embodiment.

The blood vessel extraction means 65 performs blood vessel extraction in the fourth to the sixth narrowband image data obtained by the CCD 44.

This embodiment uses the differences in the hemoglobin light absorption characteristics and the digestive tract mucosa scattering characteristics among the fourth to the sixth narrowband light to extract a superficial-layer blood vessel in the fourth narrowband image; both a superficial-layer blood vessel and an intermediate-layer blood vessel in the fifth narrowband image; and an intermediate-layer blood vessel in the sixth narrowband image.

To extract signals of the frequency components respectively corresponding to a superficial-layer blood vessel and an intermediate-layer blood vessel, two different two-dimensional filters are produced to suit the purposes. The two-dimensional filters may be produced as in the first embodiment.

The superficial-layer blood vessel extraction filter thus produced is applied to the fourth and the fifth narrow band images while the intermediate-layer blood vessel extraction filter thus produced is applied to the fifth and the sixth narrowband images to produce intensified narrowband images having the superficial-layer blood vessel and the intermediate-layer blood vessel intensified, respectively.

Thus, four different intensified narrowband images are produced: a fourth superficial layer-intensified narrowband image, a fifth superficial layer-intensified narrowband image, a fifth intermediate layer-intensified narrowband image, and a sixth intermediate layer-intensified narrowband image.

Upon production of intensified narrowband images, the alignment means 66 calculates the shift amounts of the narrowband images from the intensified narrowband images (positional shifts to be corrected) to achieve alignment.

According to this embodiment, the fourth and the sixth narrowband images are aligned with the fifth narrowband image. The alignment is achieved as in the first embodiment. The shift amount of the fourth superficial layer-intensified narrowband image is calculated with respect to a reference provided by the fifth superficial-layer intensified narrowband image for superficial-layer blood vessels while the shift amount of the sixth intermediate layer-intensified narrowband image is calculated with respect to a reference provided by the fifth intermediate-layer intensified narrowband image for intermediate-layer blood vessels, whereupon the fourth narrowband image and the sixth narrowband image are moved by the respective shift amounts to obtain a fourth moved narrowband image and a sixth moved narrowband image.

This embodiment shares the procedure to follow with the first embodiment.

The third embodiment of the invention comprises a broadband light source 90 in addition to the three narrowband light sources used in the second embodiment.

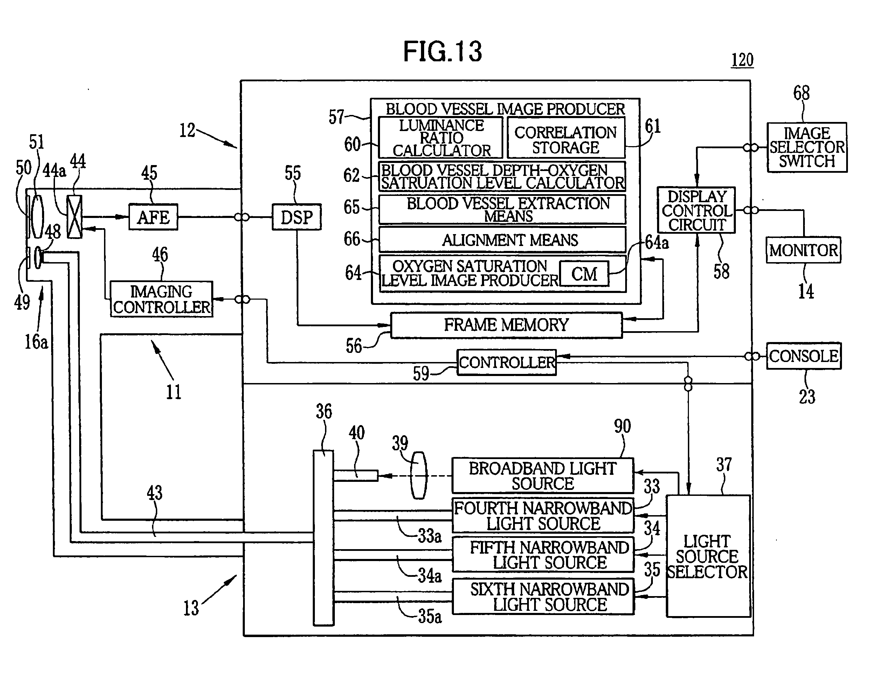

As illustrated in Fig. 13, the light source device 13 comprises the broadband light source 90, the fourth to the sixth narrowband light sources 33 to 35, the coupler 36, and the light source selector 37.

The light source 90 uses, for example, a laser diode 90a having a wavelength of 445 nm as an excitation light source, which is applied to a Micro White (trademark) fluorescent body 90b to produce the broadband light BB. The broadband light source 90 comprises the condenser lens 39 and a broadband optical fiber 40 on the front side thereof; the broadband optical fiber 40 is connected to the coupler 36.

The light source selector 37 is connected to the controller 59 in the processor and turns on or off the broadband light source 90 and the fourth to the sixth narrowband light sources 33 to 35 according to an instruction by the controller 59. According to the third embodiment, the broadband light source 90 and the fourth to the sixth narrowband light sources 33 to 35 are sequentially turned on to perform imaging using the broadband light BB and the fourth to the sixth narrowband light N4 to N6.

Similarly to the first embodiment, the broadband light BB and the fourth narrowband light N4 to the sixth narrowband light N6 enter the CCD 44 of the endoscope 11 to obtain a broadband signal and a fourth narrowband imaging signal to a sixth narrowband imaging signal.

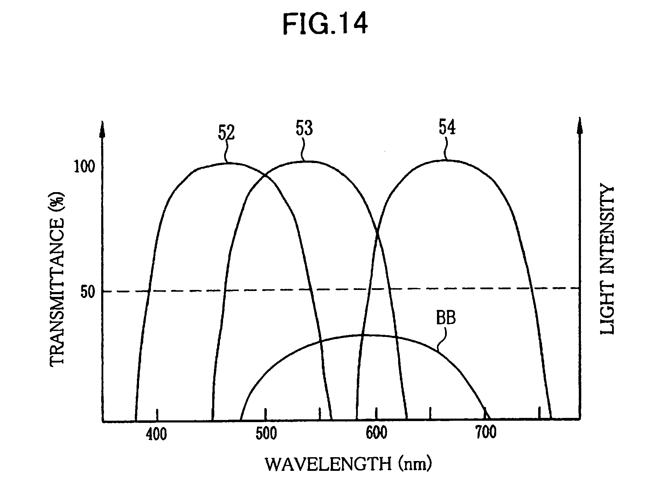

The CCD 44 comprises red (R) filters, green (G) filters, and blue (B) filters, which have spectral transmittances 52, 53, and 54, respectively as illustrated in Fig. 14. This embodiment differs from the second embodiment in that the light sources include broadband light. Among the light entering the condenser lens 51, the broadband light BB has a wavelength ranging from about 470 nm to 700 nm. Therefore, the red filters, the green filters, and the blue filters pass wavelength ranges respectively corresponding to their spectral transmittances 52, 53, 54 for the broadband light BB. Now, let imaging signal R be a signal photoelectrically converted by a red pixel, imaging signal G a signal photoelectrically converted by a green pixel, and imaging signal B a signal photoelectrically converted by a blue pixel. Then, the broadband light BB entering the CCD 44 gives a broadband imaging signal composed of the imaging signal R, the imaging signal G, and the imaging signal B. The narrowband light N4 to N6 are the same as in the second embodiment.

Imaging with the broadband light and the fourth to the sixth narrowband light N4 to N6 is performed for example using the fourth narrowband light N4, the fifth narrowband light N5, and the sixth narrowband light N6 in this order to store the fourth narrowband image data, the fifth narrowband image data, and the sixth narrowband image data in the frame memory 56.

The differences between the first and the second embodiments are the configuration of the light source 13, the blood vessel extraction processing by the blood vessel extraction means 65, and the alignment means 66. The first and the second embodiments share the other features, and their descriptions therefore will not be repeated below.

When the broadband image data and the fourth to the sixth narrowband image data have been obtained, the blood vessel extraction means 65 performs blood vessel extraction processing.

In the blood vessel extraction processing, two different two-dimensional filters are produced, i.e., a superficial-layer blood vessel extraction filter and an intermediate-layer blood vessel extraction filter, to extract frequencies corresponding respectively to a superficial-layer blood vessel and an intermediate-layer blood vessel and applied to the broadband image data and the fourth to the sixth narrowband image data.

The superficial-layer blood vessel extraction filter thus produced is applied to the broadband image and the fourth narrow band images while the intermediate-layer blood vessel extraction filter thus produced is applied to the broadband image and the fifth and the sixth narrowband images to produce blood vessel-intensified narrowband images having the superficial-layer blood vessel and the intermediate-layer blood vessel intensified.

Thus, five different blood vessel-intensified images are produced: a fourth superficial layer-intensified narrowband image, a superficial layer-intensified broadband image, an intermediate layer-intensified broadband image, a fifth intermediate layer-intensified narrowband image, and a sixth intermediate layer-intensified narrowband image.

Upon production of five different blood vessel-intensified narrowband images, the alignment means 66 calculates the shift amounts of the narrowband images from the blood vessel-intensified images to achieve alignment.

According to this embodiment, the fourth to the sixth narrowband images are aligned with the broadband image. The alignment is achieved as in the first embodiment: the shift amount of the fourth superficial layer-intensified narrowband image is calculated in respect of superficial-layer blood vessels with respect to the superficial-layer intensified broadband image used as reference while,the shift amounts of the fifth and the sixth intermediate layer-intensified narrowband images are calculated in respect of intermediate-layer blood vessels with respect to the superficial-layer intensified broadband image used as reference, whereupon the fourth narrowband image to the sixth narrowband image are moved by their respective shift amounts to obtain the fourth to the sixth moved narrowband images.

This embodiment shares the procedure to follow with the first embodiment.

The present invention is basically as described above. Note that various improvements and modifications may be made without departing from the scope of the claims.

The endoscope system according to the present invention, which is of course not limited to the first to the third embodiments, may be applied to both still images and moving images.

## Claims

1. An electronic endoscope system comprising:
a light source device (30) for sequentially emitting plural kinds of light having different wavelength bands from each other;
an electronic endoscope (11) for sequentially illuminating the plural kinds of light emitted from said light source device to a subject tissue containing blood vessels inside a body cavity, sequentially receiving the plural kinds of reflected light of the illuminated light from the subject tissue, and sequentially outputting image data corresponding to the plural kinds of received light having the different wavelength bands;
**characterised by** blood vessel extraction means (65) for extracting positions of a blood vessel having a specified diameter from each of image data corresponding to the plural kinds of light having different wavelength bands outputted from said electronic endoscope; and by
alignment means for aligning images corresponding to the image data obtained using the plural kinds of light having different wavelength bands based on the positions of the blood vessel extracted by said blood vessel extraction means,
image production means (64), for producing an oxygen saturation level image representing a distribution of an oxygen saturation level in the blood vessel from the image data of the images aligned by said alignment means; and
image display means (14) for displaying in simulated color an oxygen saturation level image produced by said image production means.

2. The electronic endoscope system according to Claim 1,
wherein said light source device sequentially emits three kinds of light having the different wavelength bands, and
wherein said alignment means aligns, with respect to an image of image data obtained using a kind of light having an intermediate wavelength band of the three kinds of light having different wavelength bands, other images of image data obtained using two kinds of light having other wavelength bands of the three kinds of light.

3. The electronic endoscope system according to Claim 1,
wherein said light source device sequentially emits three kinds of light having different wavelength bands,
wherein said blood vessel extraction means extracts positions of a first blood vessel having a first diameter from first image data among the image data corresponding to the three kinds of light having different wavelength bands, extracts positions of the first blood vessel and positions of a second blood vessel having a second diameter greater than the first diameter from second image data among the image data, and extracts positions of the second blood vessel from third image data among the image data, and
wherein said alignment means aligns two images of the first and the second image data based on the positions of the first blood vessel, and aligns two images of the second and the third image data based on the positions of the second blood vessel.

4. The electronic endoscope system according to Claim 1,
wherein said light source device sequentially emits as a first light the three kinds of light having different wavelength bands and as a second light broadband light containing light having a whole wavelength band of the first light, and
wherein said alignment means aligns each of images of individual image data obtained using the first light with an image of image data obtained using the second light.

5. The electronic endoscope system according to Claim 1 or 2, wherein said light source device sequentially emits three kinds of light having wavelengths of 540±10nm, 560±10nm and 580±10nm.

6. The electronic endoscope system according to any one of Claims 1 to 4, wherein said light source device sequentially emits three kinds of light having wavelengths of 405±10nm, 440±10nm and 470±10nm.

## Patentansprüche

1. Elektronisches Endoskopsystem, umfassend:
eine Lichtquelleneinrichtung (30) zum sequentiellen Emittieren mehrerer Arten von Licht, die voneinander verschiedene Wellenlängenbänder haben;
ein elektronisches Endoskop (11) zum sequentiellen Beleuchten eines Blutgefäße enthaltenden Subjektgewebes im Inneren eines Körperhohlraums mit den mehreren Arten von Licht, die von der Lichtquelleneinrichtung emittiert werden, zum sequentiellen Empfangen der mehreren Arten reflektierten Lichts des Beleuchtungslichts von dem Subjektgewebe, und zum sequentiellen Ausgeben von Bilddaten entsprechend den mehreren Arten empfangenen Lichts mit den verschiedenen Wellenlängenbändern;
**gekennzeichnet durch**
Blutgefäß-Extraktionsmittel (65) zum Extrahieren von Stellen eines Blutgefäßes mit einem spezifizierten Durchmesser aus sämtlichen Bilddaten entsprechend den mehreren Arten von Licht mit verschiedenen Wellenlängenbändern, die von dem elektronischen Endoskop ausgegeben werden;
Ausrichtmittel (66) zum Ausrichten von Bildern entsprechend den unter Verwendung der mehreren Arten von Licht verschiedener Wellenlängenbänder gewonnenen Bilddaten, basierend auf den Stellen des Blutgefäßes, die von den Blutgefäß-Extrahiermitteln extrahiert wurden,
Bilderzeugungsmittel (64) zum Erzeugen eines Sauerstoff-Sättigungsspiegelbilds, welches eine Verteilung eines Sauerstoff-Sättigungsspiegels in dem Blutgefäß repräsentiert, aus den Bilddaten der von den Ausrichtmitteln ausgerichteten Bilder; und
Bildanzeigemittel (14) zum Anzeigen eines von den Bilderzeugungsmitteln erzeugten Sauerstoff-Sättigungsspiegelbilds in simulierter Farbe.

2. Elektronisches Endoskopsystem nach Anspruch 1,
bei dem die Bildquelleneinrichtung sequentiell drei Arten von Licht mit den verschiedenen Wellenlängenbändern emittiert, und
wobei die Ausrichtmittel bezüglich eines Bilds von Bilddaten, die unter Verwendung einer Art von Licht mit einem Zwischenwellenlängenband der drei Arten von Licht mit verschiedenen Wellenlängenbändern erhalten wurden andere Bilder von Bilddaten ausrichtet, die unter Verwendung von zwei Arten von Licht mit anderen Wellenlängenbändern der drei Arten von Licht gewonnen wurden.

3. Elektronisches Endoskopsystem nach Anspruch 1,
bei dem die Lichtquelleneinrichtung sequentiell drei Arten von Licht unterschiedlicher Wellenlängenbänder emittiert,
wobei die Blutgefäß-Extrahiermittel Stellen eines ersten Blutgefäßes mit einem ersten Durchmesser aus ersten Blutdaten unter den Bilddaten entsprechend den drei Arten von Licht mit verschiedenen Wellenlängenbändern extrahiert, Stellen des ersten Blutgefäßes und Stellen eines zweiten Blutgefäßes mit einem zweiten Durchmesser größer als der erste Durchmesser aus zweiten Bilddaten unter den Bilddaten extrahiert, und Stellen des zweiten Blutgefäßes aus dritten Bilddaten unter den Bilddaten extrahiert, und
wobei die Ausrichtmittel zwei Bilder der ersten und der zweiten Bilddaten basierend auf den Stellen des ersten Blutgefäßes ausrichtet, und zwei Bilder der zweiten und der dritten Bilddaten basierend auf den Stellen des zweiten Blutgefäßes ausrichtet.

4. Elektronisches Endoskopsystem nach Anspruch 1, bei dem die Lichtquelleneinrichtung sequentiell als erstes Licht der drei Arten von Licht mit verschiedenen Wellenlängenbändern und als ein zweites Licht breitbandiges Licht, das Licht mit einem Gesamtwellenlängenband des ersten Lichts enthält, emittiert, und
wobei die Ausrichtmittel jedes der Bilder individueller Bilddaten, die mit dem ersten Licht gewonnen wurden, ausrichtet mit einem Bild der Bilddaten, die unter Verwendung des zweiten Lichts gewonnen wurden.

5. Elektronisches Endoskopsystem nach Anspruch 1 oder 2, bei dem die Lichtquelleneinrichtung sequentiell drei Arten von Licht mit Wellenlängen von 540 ± 10 nm, 560 ± 10 nm und 580 ± 10 nm emittiert.

6. Elektronisches Endoskopsystem nach einem der Ansprüche 1 bis 4, bei dem die Lichtquelleneinrichtung sequentiell drei Arten von Licht mit Wellenlängen von 405 ± 10 nm, 440 ± 10 nm und 470 ± 10 nm emittiert.

## Revendications

1. Système d'endoscope électronique comprenant :
un dispositif de source de lumière (30) pour émettre séquentiellement plusieurs types de lumières ayant des bandes de longueur d'onde différentes les unes des autres ;
un endoscope électronique (11) pour rayonner séquentiellement la pluralité de types de lumières émises à partir dudit dispositif de source de lumière vers un tissu de sujet contenant des vaisseaux sanguins à l'intérieur d'une cavité corporelle, recevoir séquentiellement la pluralité de types de lumières réfléchies de la lumière rayonnée provenant du tissu de sujet, et délivrer séquentiellement des données d'image correspondant à la pluralité de types de lumières reçues ayant les différentes bandes de longueur d'onde ;
**caractérisé par**
des moyens d'extraction de vaisseau sanguin (65) pour extraire des positions d'un vaisseau sanguin ayant un diamètre spécifié de chacune des données d'image correspondant à la pluralité de types de lumières ayant différentes bandes de longueur d'onde délivrées à partir dudit endoscope électronique ; et par
des moyens d'alignement (66) pour aligner des images correspondant aux données d'image obtenues en utilisant la pluralité de types de lumières ayant différentes bandes de longueur d'onde sur la base des positions du vaisseau sanguin extraites par lesdits moyens d'extraction de vaisseau sanguin ;
des moyens de production d'image (64) pour produire une image de niveau de saturation d'oxygène représentant une distribution d'un niveau de saturation d'oxygène dans le vaisseau sanguin à partir des données d'image des images alignées par lesdits moyens d'alignement ; et
des moyens d'affichage d'image (14) pour afficher en une couleur simulée une image de niveau de saturation d'oxygène produite par lesdits moyens de production d'image.

2. Système d'endoscope électronique selon la revendication 1,
dans lequel ledit dispositif de source de lumière émet séquentiellement trois types de lumières ayant les différentes bandes de longueur d'onde, et
dans lequel lesdits moyens d'alignement alignent, par rapport à une image de données d'image obtenues en utilisant un type de lumière ayant une bande de longueur d'onde intermédiaire des trois types de lumières ayant différentes bandes de longueur d'onde, d'autres images de données d'image obtenues en utilisant deux types de lumières ayant d'autres bandes de longueur d'onde des trois types de lumières.

3. Système d'endoscope électronique selon la revendication 1,
dans lequel ledit dispositif de source de lumière émet séquentiellement trois types de lumières ayant différentes bandes de longueur d'onde,
dans lequel lesdits moyens d'extraction de vaisseau sanguin extraient des positions d'un premier vaisseau sanguin ayant un premier diamètre de premières données d'image parmi les données d'image correspondant aux trois types de lumières ayant différentes bandes de longueur d'onde, extraient des positions du premier vaisseau sanguin et des positions d'un deuxième vaisseau sanguin ayant un deuxième diamètre plus grand que le premier diamètre de deuxièmes données d'image parmi les données d'image, et extraient des positions du deuxième vaisseau sanguin de troisièmes données d'image parmi les données d'image, et
dans lequel lesdits moyens d'alignement alignent deux images des premières et deuxièmes données d'image sur la base des positions du premier vaisseau sanguin, et alignent deux images des deuxièmes et troisièmes données d'image sur la base des positions du deuxième vaisseau sanguin.

4. Système d'endoscope électronique selon la revendication 1,
dans lequel ledit dispositif de source de lumière émet séquentiellement, en tant que première lumière, les trois types de lumières ayant différentes bandes de longueur d'onde et, en tant que deuxième lumière, une lumière large bande contenant une lumière ayant une bande de longueur d'onde entière de la première lumière, et
dans lequel lesdits moyens d'alignement alignent chacune des images de données d'image individuelles obtenues en utilisant la première lumière avec une image de données d'image obtenues en utilisant la deuxième lumière.

5. Système d'endoscope électronique selon la revendication 1 ou 2, dans lequel ledit dispositif de source de lumière émet séquentiellement trois types de lumières ayant des longueurs d'onde de 540 ± 10 nm, 560 ± 10 nm et 580 ± 10 nm.

6. Système d'endoscope électronique selon l'une quelconque des revendications 1 à 4, dans lequel ledit dispositif de source de lumière émet séquentiellement trois types de lumières ayant des longueurs d'onde de 405 ± 10 nm, 440 ± 10 nm et 470 ± 10 nm.
